# EUROPÄISCHE PATENTSCHRIFT

(11) **EP 0 744 992 B1**
(45) Veröffentlichungstag und Bekanntmachung des Hinweises auf die Patenterteilung: **15.10.1997**
(21) Anmeldenummer: 95909720.5
(22) Anmeldetag: 14.02.1995
(51) Int. Cl.: B01J 2/00, B01J 2/04

(54) **VERFAHREN ZUR HERSTELLUNG VON PARTIKELN BZW. PULVERN**
PROCESS FOR PREPARING PARTICLES OR POWDERS
PROCEDE POUR LA FABRICATION DE PARTICULES OU DE POUDRES

(30) Priorität: 15.02.1994 SI 9400079
(43) Veröffentlichungstag der Anmeldung: 04.12.1996
(73) Patentinhaber: Weidner, Eckhard, 91056 Erlangen (DE); Knez, Zeljko, 62000 Maribor (SI); Novak, Zoran, 62000 Maribor (SI)
(72) Erfinder: Weidner, Eckhard, 91056 Erlangen (DE); Knez, Zeljko, 62000 Maribor (SI); Novak, Zoran, 62000 Maribor (SI)
(74) Vertreter: Beyer, Andreas, Dr.
(86) Internationale Anmeldenummer: EP9500538
(87) Internationale Veröffentlichungsnummer: WO9521688

(56) Entgegenhaltungen:
- EP-A- 0 036 523
- EP-A- 0 322 687
- EP-A- 0 542 314
- DATABASE WPI Derwent Publications Ltd., London, GB; AN 92021411 & JP,A,03 271 113 (KOBE STEEL KK) , 3.Dezember 1991
- DATABASE WPI Derwent Publications Ltd., London, GB; AN 89244463 & JP,A,01 176 437 (ONO PHARMACEUTICAL KK) , 12.Juli 1989

## Beschreibung

Die Erfindung betrifft ein Verfahren zur Herstellung von Partikeln bzw. Pulvern.

### Stand der Technik

Die im Rahmen chemisch-technischer Prozesse anfallenden Partikeln und Partikelgrößenverteilungen fester Stoffe sind im allgemeinen nicht diejenigen, die für die weitere Anwendung dieser Stoffe erwünscht oder erforderlich sind. Deshalb werden solche Substanzen häufig zerkleinert oder umkristallisiert. Übliche Verfahren zur Änderung von Partikelgrößen und Verteilungen sind Brechung/Mahlung, Zerstäubung/Sprühkristallisation, Gefriertrocknung, Sublimation, Rekristallisation aus Lösungen. Die Anwendung der genannten Prozesse ist mit verschiedenen verfahrenstechnischen Nachteilen verbunden. Bei den mechanischen Verfahren tritt eine teilweise erhebliche Erwärmung der behandelten Stoffe auf, die bei thermisch labilen Substanzen oder Substanzgemischen zur Zerstörung der Inhaltsstoffe führen kann. Die thermischen Verfahren, wie z.B. Sublimation oder Gefriertrocknung, sind nur für wenige Stoffe anwendbar. Bei Kristallisationsverfahren werden Lösemittel verwendet, die aus den Feststoffen nur schwer zu entfernen sind und häufig als Abfallstoff anfallen.

Es ist auch bekannt, kompressible Fluide, z.B. nahe- oder überkritische Fluide, im Rahmen der Herstellung von Partikeln bzw. Pulvern einzusetzen. Im folgenden werden hierzu drei Beispiele angegeben:

### 1. Kristallisation aus überkritischen Lösungen

Im Vergleich zur herkömmlichen Lösemittelkristallisation ist dieses Verfahren insbesondere dann von Vorteil, wenn schwerflüchtige, thermisch empfindliche Substanzen zu kristallisieren sind. Als ungiftige Lösungsmittel werden Fluide mit einer kritischen Temperatur im Bereich der Umgebungstemperatur als interessante Alternativen zu klassischen organischen Lösungsmitteln eingesetzt /1/.

Bei der konventionellen, absatzweise betriebenen Kühlungskristallisation wird eine gesättigte Lösung beginnend von einer hohen Temperatur, bei der das Lösemittel gute Lösefähigkeit hat, entlang einer optimalen Kühlungskurve auf die Endtemperatur abgekühlt. Die Lösefähigkeit wird dadurch vermindert und der gelöste Stoff fällt zumindest teilweise aus. Eine Optimierung der Kühlungskurve ist notwendig, um eine möglichst konstante Übersättigung und ein konstantes Kristallwachstum einzustellen.

Für den Fall, daß es sich bei dem Lösungsmittel um ein überkritisches Fluid handelt, in dem die zu kristallisierende Substanz aufgelöst ist, müssen Übersättigung und Kristallwachstum ebenfalls optimiert werden. Im Unterschied zur konventionellen Kristallisation steht hierbei mit dem im Kristallisationsbehälter herrschenden Druck ein weiterer Parameter zur Beeinflussung der Kristallbildung zur Verfügung. Typische Kristallisationszeiten liegen zwischen 30min und mehreren Stunden. Nach Entspannung des Behälterinhalts liegen die Kristalle in fester, lösemittelfreier Form vor.

### 2. RESS-Prozeß (Rapid Expansion of a Supercritical Solution)

Beim RESS-Verfahren wird ein Feststoff in einem überkritischen Fluid unter Druck aufgelöst und die so gebildete überkritische Lösung dann schnell auf einen niedrigeren Druck, vorzugsweise auf Atmosphärendruck entspannt. Das Lösevermögen des überkritischen Fluids wird dadurch sehr schnell vermindert und der zu kristallisierende Stoff fällt als Feststoff aus. Die Anwendbarkeit dieses Konzepts wurde für einige Substanzklassen gezeigt. Dazu gehören Polymere /2/, Farbstoffe /3/, Pharmazeutika /4/ und anorganische Substanzen /5/. Durch Variation der Prozeßbedingungen wird die Übersättigung und die Keimbildungsrate beeinflußt. Dadurch können Partikeln erhalten werden, deren Größe, Größenverteilung und Morphologie sich stark vom festen Ausgangsmaterial unterscheiden. Kennzeichen des Prozesses ist die aufgrund der Abkühlung und der starken Dichterniedrigung des überkritischen Fluids bei der Entspannung erreichte extrem hohe Übersättigung.

### 3. GASR-Prozeß (Gas Antisolvent Rekristallisation)

Diese Technik wird bevorzugt für Stoffe angewandt, die in überkritischen Medien unlöslich sind. Bei dieser Methode wird die Löslichkeit eines Gases unter Druck in einem organischen Lösungsmittel genutzt, um die Lösefähigkeit dieses organischen Lösungsmittel für darin gelöste Substanzen zu vermindern. Durch Zugabe des Gases wird eine Fällung ausgelöst. Auch bei diesem Prozeß können durch Variation von Druck, Temperatur und Gasart die Eigenschaften der Partikelkollektive in weiten Grenzen variiert werden. Ein wesentlicher Vorteil der Hochdruckverfahren - die Lösemittelfreiheit - wird jedoch beim GASR-Verfahren aufgegeben.

Die bekannten Anwendungen überkritischer Fluide zur Erzeugung von Feststoffen haben verschiedene Nachteile. Die Kristallisationsverfahren (Kristallisation aus überkritischen Fluiden und Gas Antisolventkristallisation) können nur absatzweise betrieben werden und erfordern lange Abkühl- oder Druckänderungszeiten (teilweise mehrere Stunden). Nach Beendigung der Kristallisationsphase muß der Autoklaveninhalt entspannt werden, um die festen Produkte austragen zu können. Im Fall des GASR-Prozesses fällt das Produkt nach dem Entspannen und dem damit verbundenen Abtrennen des Gases in suspendierter Form im Lösungsmittel oder als feuchter Feststoffkuchen an. Der Feststoff muß durch geeignete Maßnahmen abgetrennt und getrocknet werden. Bei der Kristallisation aus überkritischen Fluiden sind sehr hohe Drucke und große Gasmengen erforderlich, da die betreffenden Stoffe in überkritischen Fluiden häufig nur wenig löslich sind. Von Tavana und Randolph /1/ wird beispielsweise die Kristallisation von Benzoesäure aus einer Lösung in Kohlendioxid beschrieben. Bei einem Druck von 282,8 bar und einer Temperatur von 55 °C sind 2 Gew.% Benzoesäure in Kohlendioxid löslich. Unter der Voraussetzung, daß die gesamte Benzoesäure als Kristallisat gewonnen wird, müssen demnach für 1 kg Produkt 49 kg Gas auf die Kristallisationsendtemperatur von 35 °C abgekühlt werden. Zusätzlich zum thermischen Energiebdarf werden erhebliche Energiemengen zur mechanischen Rekompression der großen Gasmengen benötigt.

Ähnliche Gesichtspunkte gelten auch für den RESS-Prozeß. Auch hier sind sehr hohe Drücke von z.T. über 600 bar /5/ und große Gasüberschüsse erforderlich, um Feststoffe im überkritischen Fluid aufzulösen. Es werden Verfahren beschrieben, die mehrere Hundert Kilogramm Gas zur Gewinnung von 1 kg Pulver erfordern.

Aus der Gasextraktion ist ebenfalls bekannt, daß überkritische Medien für sehr viele Substanzen ein schlechtes Lösevermögen aufweisen. Sowohl bei der Gasextraktion als auch bei den oben beschriebenen Verfahren zur Partikelerzeugung sind deshalb sehr hohe Drücke und große Lösemittelmengen erforderlich.

### Beschreibung der Erfindung

Der Erfindung liegt die Aufgabe zugrunde, ein Verfahren bereitzustellen, das die oben genannten Nachteile der klassischen Verfahren und der Hochdruckverfahren vermeidet.

Diese Aufgabe ist erfindungsgemäß durch ein Verfahren gelöst, das die im Anspruch 1 genannten Schritte umfaßt. Bevorzugte Ausgestaltungen und Weiterbildungen des erfindungsgemäßen Verfahrens sind in den Unteransprüchen angegeben.

Dem erfindungsgemäßen Verfahren liegt die überraschende Erkenntnis zugrunde, daß es zur Erzeugung von Partikeln nicht erforderlich ist, von überkritischen Lösungen auszugehen. Vielmehr genügt es völlig, Gase oder allgemein kompressible Fluide in der zu behandelnden Substanz aufzulösen. Die so entstehende Lösung, die bevorzugt mit kompressiblem Fluid gesättigt ist, wird in einer geeigneten Entspannungsvorrichtung schnell entspannt. Sofern es sich bei dem verwendeten kompressiblen Fluid um ein Gas handelt, entweicht dieses beim Entspannen und bewirkt eine Abkühlung, die so groß ist, daß die Erstarrungstemperatur der zu behandelnden Substanz unterschritten wird. Die Substanz fällt in feinteiliger Form aus und wird vom Gasstrom mit geeigneten Verfahren (z.B. Sedimentation, Zyklon, Filtration, Elektrofiltration) abgetrennt und, falls gewünscht, fraktioniert. Aufgrund der soeben beschriebenen Vorgehensweise wird das erfindungsgemäße Verfahren im folgenden auch als PGSS-Prozeß (Particles from Gas Saturated Solutions) bezeichnet.

Die zu behandelnde Substanz kann bei Raumbedingungen entweder ein Feststoff oder eine Flüssigkeit sein. Liegt die zu behandelnde Substanz oder das zu behandelnde Substanzgemisch als Feststoff vor, so wird durch Auflösen des kompressiblen Fluids eine unter Druck stehende flüssige Lösung erzeugt. Das Massenverhältnis zwischen dem kompressiblen Fluid und der zu behandelnden Substanz liegt dabei zwischen 0,1 : 1 und 4 : 1 und ist damit um 2 bis 3 Größenordnungen geringer als bei den anderen Hochdruckverfahrenstechniken zur Feststofferzeugung.

Die Löslichkeit von Gasen in Flüssigkeiten ist wesentlich größer als diejenige von Flüssigkeiten/Feststoffen in Gasen. So beträgt die Löslichkeit von Stearinsäure in Ethan bei einer Temperatur von 80 °C und einem Druck von 37 bar 0,002 Gew.%. Bei gleichen Druck- und Temperaturbedingungen beträgt die Löslichkeit von Ethan in Stearinsäure aber bereits 5,62 Gew.%. Bei der Entspannung der gashaltigen flüssigen Lösung aus beispielsweise Ethan in Stearinsäure in einer geeigneten Vorrichtung, z.B einer handelsüblichen Hochdruckdüse, geht das komprimierte Fluid, hier Ethan, wieder in den gasförmigen Zustand über. Überraschenderweise und für den Fachmann nicht vorhersehbar wurde nun festgestellt, daß die Abkühlung trotz des ungewöhnlich niedrigen Gasanteils so stark ist, daß die Erstarrungstemperatur des zu behandelnden Stoffes nach der Entspannungsvorrichtung unterschritten wird. Der zu behandelnde Stoff fällt deshalb als feinteiliger Feststoff aus. Damit die Erstarrungstemperatur erreicht wird, sind bestimmte Randbedingungen bezüglich der Temperaturen einzuhalten, bei denen das kompressible Fluid aufgelöst wird. Der Richtwert für die Temperatur vor der Entspannung soll im Falle von Substanzen oder Substanzmischungen, die eine klar definierte Schmelztemperatur haben, im Bereich von +/-50 K, vorzugsweise +/-20 K über oder unter dem Schmelzpunkt bei Atmosphärendruck liegen. Mittels des erfindungsgemäßen Verfahrens entstehen Lösungen einer Substanz oder eines Substanzgemisches auch bei Temperaturen unterhalb des Schmelzpunktes, den diese Substanz bzw. Substanzmischung bei Normalbedingungen, d.h. bei Atmosphärendruck hat. Offensichtlich wird also der Schmelzpunkt einer Substanz oder eines Substanzgemisches durch das Auflösen eines kompressiblen Fluids unter Druck herabgesetzt. So wurde beispielsweise festgestellt, daß der Schmelzpunkt von Glycerin-1-Stearinsäure-ester, der bei Atmosphärendruck bei 75 °C liegt, in einer Kohlendioxidatmosphäre von 150 bar auf 58 °C zurückgeht. Unter Propanatmosphäre wird ein Schmelzpunkt von 58 °C bereits bei einem Druck von 20 bar erreicht.

Diese Tatsache ist von besonderer Bedeutung bei der Behandlung von Substanzen, die sich bereits vor Erreichen des Schmelzpunktes zersetzen. Durch Auswahl eines geeigneten kompressiblen Fluids können erfindungsgemäß flüssige Lösungen bei Temperaturen erreicht werden, die deutlich unterhalb des Zersetzungspunktes liegen.

In einer weiteren Ausführungsform des erfindungsgemäßen Verfahrens wird der Schmelzpunkt einer Substanz durch Zugabe eines inkompressiblen Hilfsstoffs abgesenkt. Der inkompressible Hilfsstoff wird dabei so ausgewählt, daß er mit der zu versprühenden Substanz ein niedrigschmelzendes Eutektikum bildet. In der so gebildeten eutektischen Mischung wird dann in der oben beschriebenen Weise ein kompressibles Fluid aufgelöst und die entstandene flüssige Lösung schnell entspannt. Die eutektische Mischung erstarrt aufgrund der Abkühlung nach der Entspannung.

Als kompressible Fluide kommen eine ganze Reihe von Substanzen in Betracht. In einer bevorzugten Ausführungsform werden Kohlendioxid, kurzkettige Alkane, Distickstoffmonoxid, Stickstoff allein oder in Mischungen eingesetzt. Im Prinzip kann aber die Dampfphase jedes der in Tabelle 1 genannten Stoffe und Mischungen dieser Stoffe als kompressibles Fluid verwendet werden.

**Tabelle 1**

| Verbindung | Siedepunkt [°C] | Kritische Temperatur [°C] | Kritischer Druck [bar] | Kritische Dichte [kg/m³] |
|---|---|---|---|---|
| CO₂ | -78,5 | 31,3 | 72,9 | 0,448 |
| NH₃ | -33,35 | 132,4 | 112,5 | 0,235 |
| H₂O | 100,00 | 374,15 | 218,3 | 0,315 |
| N₂O | -88,56 | 36,5 | 71,7 | 0,45 |
| CH₄ | -164,00 | -82,1 | 45,8 | 0,2 |
| Ethan | -88,63 | 32,28 | 48,1 | 0,203 |
| Ethylen | -103,7 | 9,21 | 49,7 | 0,218 |
| Propan | -42,1 | 96,67 | 41,9 | 0,217 |
| Propylen | -47,4 | 91,9 | 45,4 | |
| n-Butan | -0,5 | 152,0 | 37,5 | |
| i-Butan | -11,7 | 134,7 | 35,9 | |
| n-Pentan | 36,1 | 196,6 | 33,3 | 0,232 |
| Benzol | 80,1 | 288,9 | 48,3 | 0,302 |
| Methanol | 64,7 | 240,5 | 78,9 | 0,272 |
| Ethanol | 78,5 | 243,0 | 63,0 | 0,276 |
| Isopropanol | 82,5 | 235,3 | 47,0 | 0,273 |
| Isobutanol | 108,0 | 275,0 | 42,4 | 0,272 |
| Chlorotrifluoromethan | -31,2 | 28,0 | 38,7 | 0,579 |
| Monofluormethan | 78,4 | 44,6 | 58,0 | 0,3 |
| Toluol | 110,6 | 320,0 | 40,6 | 0,292 |
| Pyridin | 115,5 | 347,0 | 55,6 | 0,312 |
| Cyclohexan | 80,74 | 280,0 | 40,2 | 0,273 |
| Cyclohexanol | 155,65 | 391,0 | 25,8 | 0,254 |
| o-Xylol | 144,4 | 357,0 | 35,0 | 0,284 |

Mit dem erfindungsgemäßen PGSS-Verfahren ist eine besonders interessante und universelle Alternative zu herkömmlichen Prozessen zur Partikelherstellung geschaffen werden. Die Hauptvorteile gegenüber konventionellen Verfahren sind:
- erheblich niedrigere Drücke als bei der Kristallisation aus überkritischen Lösungen oder beim RESS-Prozeß
- hervorragende Flexibilität und wesentlich geringerer Gasbedarf aufgrund der guten Löslichkeit der kompressiblen Fluide in Flüssigkeiten; typisch sind 0,1 - 1 kg Gas pro Kilogramm erzeugter Feststoff, wobei dieser Wert auch im Vergleich zu klassischen Verfahren der Feststofferzeugung, z.B der Sprühtrocknung, Sprühkristallisation und Tieftemperaturvermahlung, ungewöhnlich niedrig ist, denn bei den soeben genannten Prozessen werden Gase in Form von Trocknungsmedien oder als Kühlmedium benötigt. Typische Gasverbrauchszahlen liegen deshalb dort zwischen 2 und 20 kg Gas/kg Feststoff.
- Möglichkeit zur Kreislaufführung des Gases nach erfolgter Abscheidung der Feststoffe
- keine zu entsorgenden Abfallströme oder Restlösemittel
- die erzeugten Feststoffpartikeln sind lösemittelfrei
- der PGSS-Prozeß kann erfolgreich für Produkte angewandt werden, die mit anderen Verfahren nicht pulverisiert werden können (z.B. Wachse und Harze oder auch Polymerverbindungen mit ungewöhnlichen rheologischen Eigenschaften)
- der Prozeß ist für thermisch empfindliche Substanzen geeignet, da bei niedrigen Temperaturen gearbeitet werden kann
- das Verfahren ist auch zur Pulverisierung von Substanzgemischen geeignet; die Temperatur, bei der die Auflösung des Gases erfolgt, kann durch eine gezielte Auswahl von inkompressiblen Hilfsstoffen in sehr weiten Grenzen beeinflußt werden
- Staubexplosionen werden vermieden, wenn Inertgase als kompressible Medien verwendet werden.

Bevorzugte Ausführungsbeispiele des erfindungsgemäßen Verfahrens werden im folgenden auch unter Bezugnahme auf die beigefügten Zeichnungen näher erläutert. Es zeigt:
- Fig. 1: eine schematische Darstellung einer zur Durchführung des erfindungsgemäßen Verfahrens geeigneten Vorrichtung, und
- Fig. 2: eine bei einem Versuch (siehe Beispiel 4) erhaltene Partikelgrößenverteilung.

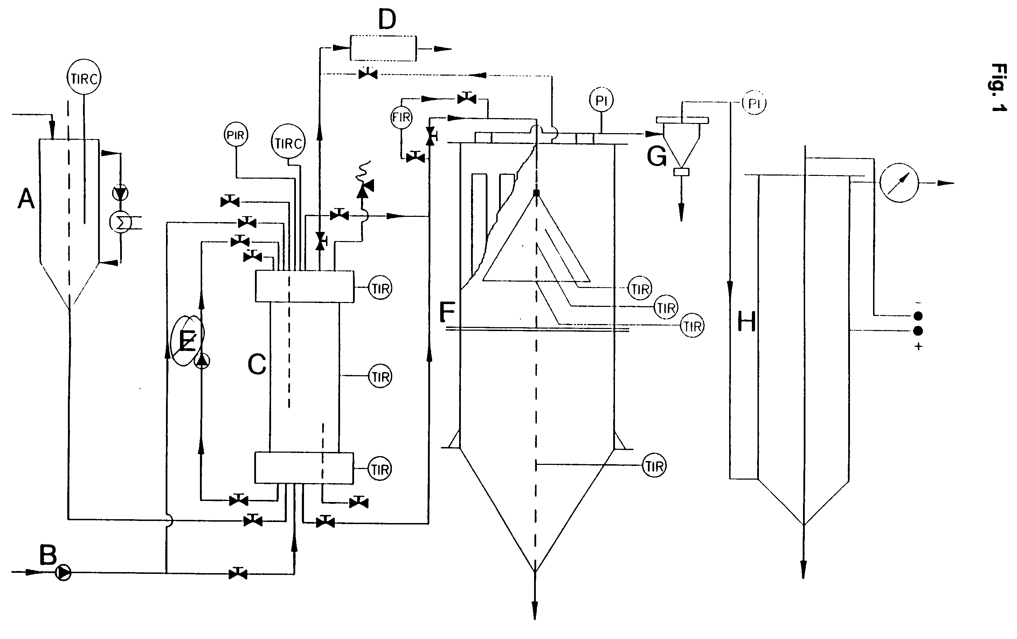

Eine zur Durchführung des erfindungsgemäßen Verfahrens geeignete Vorrichtung wird anhand von Fig. 1 näher erläutert. Eine zu behandelnde Substanz oder ein zu behandelndes Substanzgemisch wird in einem Feedgefäß A aufgeschmolzen. Ein Autoklav C (V=5 Liter, pₘₐₓ=400 bar, Tₘₐₓ=250 °C) wird vor Versuchsbeginn evakuiert. Anschließend wird die aufgeschmolzene Substanz- oder Substanzmischung eingesaugt. Das kompressible Fluid wird mit einer hier druckluftangetriebenen Hochdruckpumpe B bis zum gewünschten Druck in den Autoklaven gefördert. Der Druck wird mit einem Analogmanometer (0-600 bar) gemessen.

Mittels einer Hochdruckkreislaufpumpe E wird die flüssige Phase am Boden des Autoklaven abgezogen und zum Kopf des Autoklaven C gefördert. Durch die Flüssigphasenumwälzung wird der Stoffaustausch zwischen der flüssigen und gasförmigen Phase intensiviert; die Lösegeschwindigkeit des Gases in der Flüssigkeit wird erhöht. Während der Auflösung des Gases werden Druck und Temperatur gegebenenfalls manuell über Regelkreise korrigiert. Der Autoklav ist mit Probenahmevorrichtungen versehen, mit deren Hilfe der Gasgehalt in der flüssigen Phase gemessen werden kann. Ist der gewünschte Gasgehalt erreicht, wird der Sprühvorgang eingeleitet. Dazu wird die gashaltige Flüssigkeit über eine thermostatisierte Leitung dem Kopf eines Sprühturms F zugeführt, der zuvor mittels einer Vakuumpumpe D evakuiert und/oder mit Inertgas (z.B CO₂, N₂) gespült worden ist, um die Gefahr von Staubexplosionen mit Luftsauerstoff auszuschließen. Die gashaltige Flüssigkeit wird über eine geeignete Entspannungsvorrichtung, z.B eine Hochdruckdüse, entspannt. Alternativ können auch andere Entspannungsorgane (Handventil, Regelventil, Kapillaren, Blenden usw.) eingesetzt werden. In einer weiteren Ausführungsform kann unmittelbar vor der Düse oder in der Düse ein zusätzlicher Gasstrom zudosiert werden. Auf diese Weise können kleinere Partikelgrößen erreicht werden.

Der dem Sprühturm zugeführte Massenstrom an gashaltiger Flüssigkeit kann mittels eines Massendurchflußgerätes nach dem Coriolisprinzip gemessen werden. Um eine Druckabsenkung im Autoklaven während des Sprühvorgangs zu vermeiden, wird frisches, vorgewärmtes Gas am Kopf des Autoklaven mit Hilfe der Hochdruckpumpe B zudosiert.

Bei der Entspannung der das komprimierte Fluid enthaltenden Lösung wird das kompressible Fluid in den gasförmigen Zustand überführt. Dadurch kühlt sich das Gemisch aus kompressiblem Fluid und zu pulverisierender Substanz- bzw. Substanzmischung ab und Feststoff fällt aus. Die Temperatur und die Temperaturverteilung im Sprühturm kann mit verschiebbaren Thermoelementen gemessen werden. Der Sprühturm ist so dimensioniert, daß bevorzugt Partikel mit einem Äquivalentdurchmesser von 10 µm durch Sedimentation abgeschieden werden. Die Partikeln werden in einem Austragsgefäß aufgefangen oder können mit einer geeigneten Vorrichtung (Schleuse, Schnecke, Wirbelbett mit Überlauf u.a.) kontinuierlich ausgetragen werden. Der Sprühturm kann mit Sichtfenstern zur Beobachtung des Sprühvorgangs versehen sein.

Der von den größeren Partikeln befreite Gasstrom verläßt den Sprühturm am oberen Ende und wird einem Zyklon G zugeführt. Der Zyklon ist so dimensioniert, daß bevorzugt Partikeln mit einer Größe über 1 µm abgeschieden werden. Die Partikeln werden in einem am unteren Ende des Zyklons befestigten Austragsgefäß aufgefangen.

Zur Abtrennung von Partikeln unter 1 µm wird der den Zyklon verlassende Gasstrom in einem Elektrofilter H durch ein elektrisches Feld geführt. Die Versorgungsspannung beträgt 20 kV. Die Teilchen werden an einem Zentraldraht abgeschieden und in regelmäßigen Abständen abgerüttelt. Alternativ zum Elektrofilter können auch andere Feinfilter (z.B Gewebefilter u.ä.) eingesetzt werden.

Das Restgas wird über ein Volumenstrommeßgerät aus der Anlage herausgeführt und kann rekomprimiert und dem Autoklaven erneut zugeführt werden. Gegebenenfalls kann das Gas auch mittels eines Gebläses aus der Anlage kontinuierlich abgesaugt werden.

Bei der beschriebenen Anlage und Betriebsweise handelt es sich um eine mögliche Ausführungsform des Verfahrens. Auf einige weitere Ausführungsformen und Modifikationen wurde hingewiesen. Weitere technisch relevante Alternativen umfassen vor allem die Erzeugung der gewünschten Lösung aus kompressiblem Fluid und zu behandelnder Substanz bzw. Substanzmischung. Hier kann anstatt des Autoklaven C z.B ein statischer Mischer eingesetzt werden, in dem der Stoffaustausch zwischen Flüssigkeit und kompressiblem Fluid besonders effektiv ist. Bei Verwendung eines statischen Mischers kann das Verfahren kontinuierlich betrieben werden.

### Beispiele

### Beispiel 1 :

Glyceridmischungen aus Palmkernöl, die Ausgangsprodukte zur Herstellung von Emulgatoren und Detergentien sind, werden unter Anwendung von Propan versprüht. Der Schmelzpunkt des Produktes, das zu 60 % aus Monogylceriden, 37 % aus Diglyceriden und 2 % aus Triglyceriden und 1 % aus freien Festtsäuren besteht, beträgt 44 °C. Das Produkt wird in einem Autoklav bei einer Temperatur von 45 °C und einem Druck von 260 bar mit Propan gesättigt und über eine Düse versprüht. Die freie Fallhöhe nach der Düsenaustrittsöffnung beträgt 0,25 m. Es wird ein feinkörniges Pulver mit einer mittleren Partikelgröße von 10,5 µm erhalten. Die Temperatur des Pulvers unmittelbar nach dem Versprühen beträgt 0 °C. Die Schüttdichte des Pulvers beträgt 80 g/l.

### Beispiel 2 :

Die Mischung aus Beispiel 1 bildet bei einer Temperatur von 37 °C (die 7 °C unter dem Schmelzpunkt bei Atmosphärenbedingungen liegt) mit Propan unter einem Druck von 230 bar eine flüssige Lösung. Die propanhaltigen (ca. 35 Gew.%) Glyceride werden über eine Düse versprüht. Die Fallhöhe nach der Düsenaustritts öffnung beträgt 1,0 m. Die mittlere Partikelgröße beträgt 9,5 µm. Die Temperatur nach der Entspannung liegt bei -3 °C. Die Schüttdichte des erhaltenen Pulvers beträgt 55 g/l.

### Beispiel 3 :

In der Glyceridmischung aus Beispiel 1 wird bei einer Temperatur von 47 °C und einem Druck von 20 bar Propan aufgelöst. Der Propangehalt beträgt 22 Gew.%. Die so gebildete flüssige Lösung wird über eine Düse versprüht. Die Fallhöhe nach der Düsenaustrittsöffnung beträgt 1,0 m. Die mittlere Partikelgröße liegt bei 25 µm. Die Temperatur nach der Entspannung liegt bei 30 °C.

### Beispiel 4 :

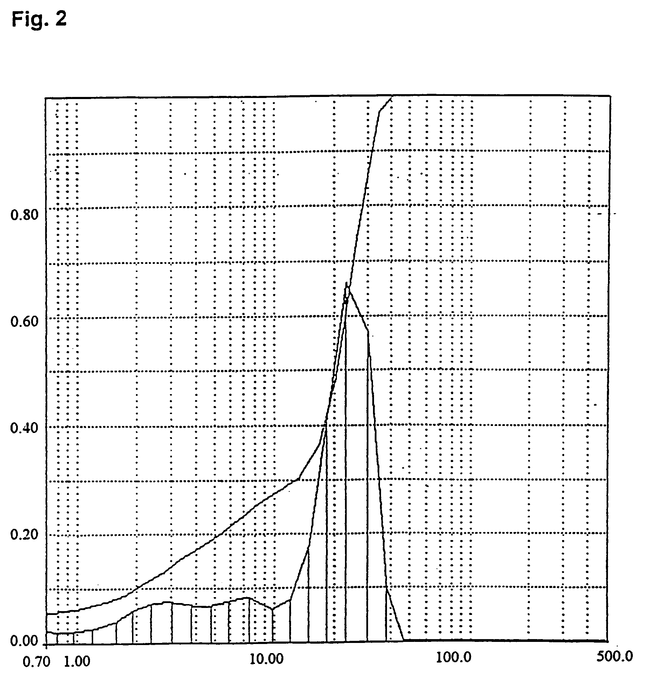

Monoglyceride der Stearinsäure werden in der Lebensmitteltechnik in großem Umfang als physiologisch unbedenklicher, hochwirksamer Emulgator eingesetzt. Es wird ein Glycerin-1-Stearinsäureester mit einem Schmelzpunkt von 75 °C und einer Reinheit von 99 Gew.% versprüht. Im Monostearat wird bei einer Temperatur von 85 °C Kohlendioxid unter einem Druck von 80 bar aufgelöst. Das Massenverhältnis zwischen Kohlendioxid und Monostearat liegt bei 0,18:1. Nach dem Versprühen über eine Düse (Fallhöhe ca. 1,8 m) wird ein feinkörniges weißes Pulver mit einer mittleren Partikelgröße von 12,8 µm und einer Schüttdichte von 39 g/l erhalten. Aus dem Zyklon wird ein Pulver mit einer mittleren Partikelgröße von 7,8 µm abgezogen. Im Elektrofilter werden wenige mg Partikel mit einer Partikelgröße < 1 µm abgeschieden. Partikelgröße und Partikelgrößenverteilung des aus dem Sprühturm abgezogenen Pulvers sind in Fig. 2 und die Zahlenwerte in Tab. 2 gezeigt.

**Tabelle 2**

| | | | | | | | | | | |
|---|---|---|---|---|---|---|---|---|---|---|
| Durchmesser µm | 0,7 | 0,9 | 1,0 | 1,4 | 1,7 | 2,0 | 2,6 | 3,2 | 4,0 | 5,0 |
| Massensummen anteil % | 5,2 | 5,9 | 6,2 | 7,5 | 8,7 | 10,2 | 13,0 | 15,4 | 17,8 | 20,1 |
| Durchmesser µm | 6,0 | 8,0 | 10,0 | 12,0 | 15,0 | 18,0 | 23,0 | 30,0 | 36,0 | 45,0 |
| Massensummen anteil % | 22,2 | 26,0 | 28,1 | 30,3 | 36,5 | 48,2 | 73,4 | 97,1 | 100 | 100 |
| Durchmesser µm | 56,0 | 70,0 | 90,0 | 110 | 135 | 165 | 210 | 260 | 320 | 400 |
| Massensummen anteil % | 100 | 100 | 100 | 100 | 100 | 100 | 100 | 100 | 100 | 100 |

### Beispiel 5 :

Zitronensäure hat bei Atmosphärendruck einen Schmelzpunkt von 156 °C. Zitronensäure wird im Massenverhältnis 1:3 mit Polyethylenglykol mit einem mittleren Molekulargewicht von 1500 g/mol und einem Schmelzbereich von 44-48 °C gemischt. Die so erzeugte Mischung schmilzt in einem Temperaturbereich zwischen von +2 bis +5 °C, d.h. die beiden Substanzen bilden ein Eutektikum mit einem Schmelzpunkt unterhalb der Schmelzpunkte der beiden Reinstoffe. In der Mischung aus Zitronensäure und Polyethylenglykol wird in einem Druckbehälter bei einer Temperatur von 20 °C Kohlendioxid unter einem Druck von 200 bar aufgelöst. Die so gebildete gashaltige Lösung wird in einer Düse entspannt. Es fällt ein pulverförmiges Koprezipitat aus Zitronensäure und Polyethylenglykol mit einer Temperatur von -5 °C an. Die mittlere Partikelgröße beträgt 300 µm. Durch Zugabe eines geeigneten Hilfsstoffs kann demnach die Temperatur, bei der das gewünschte Produkt (in diesem Fall Zitronensäure) mit Gas gesättigt werden soll, erheblich unter die Schmelztemperatur des Produkts bei Atmosphärenbedingungen abgesenkt werden.

### Beispiel 6 :

In einem Polyether mit einem mittleren Molekulargewicht von 3500 g/mol und einem Schmelzpunkt von 42 °C wird bei einer Temperatur von 45 °C Kohlendioxid unter einem Druck von 200 bar aufgelöst. Die gashaltige Lösung wird über ein manuell betätigtes Dosierventil in ein Auffanggefäß entspannt. Die Fallhöhe bis zum Boden des Auffanggefäßes beträgt etwa 0,4 m. Es wird ein Pulver mit einer breiten Partikelgrößenverteilung zwischen 200 µm und 2000 µm erhalten. Die Fraktion über 1 mm wurde abgesiebt und erneut dem Prozeß zugeführt.

### Beispiel 7 :

Ein pharmazeutischer Wirkstoff (1,4-dihydro-2,6-dimethyl-4-(2-nitrophenyl)-3,5-pyridin-Carboxylsäure, Trivialname: Nifedipin) wurde mit dem PGSS-Verfahren behandelt. Bei einer Temperatur von 145 °C und einem Druck von 200 bar wurde Kohlendioxid in dem Produkt aufgelöst. Durch Versprühung mittels einer Düse wird ein Pulver mit einer mittleren Partikelgröße von 10 µm erhalten. Das Massenverhältnis Gas zu Feststoff beträgt im vorliegenden Fall 0,1:1.

### Beispiel 8 :

Im Glycerin-1-Stearinsäureester wird bei einer Temperatur von 85 °C Kohlendioxid unter einem Druck von 80 bar aufgelöst. Das Massenverhältnis zwischen Kohlendioxid und Monostearat liegt bei 0,18:1. Unmittelbar vor dem Versprühen über eine Düse wird der gashaltigen Flüssigkeit Stickstoff zudosiert. Das Verhältnis der Massenströme Stickstoff zu gashaltiger Flüssigkeit beträgt 0,5:1. Nach der Entspannung in der Düse (Fallhöhe ca. 1,8 m) wird ein feinkörniges weißes Pulver mit einer mittleren Partikelgröße von 8,2 µm erhalten. Dieses Beispiel entspricht im wesentlichen Beispiel 4. Durch Zudosierung des Fremdgasstroms werden jedoch feinere Partikel erhalten.

### Literatur :

/1/ Tavana A., Randolph A.D., Aiche Journal 1989, 35(10) 1625
/2/ Bush P.J, Pradhan D., Ehrlich P., Macromolecules 1991, 24(6) 1439
/3/ Chang C.J., Randolph A.D., Aiche Journal 1990, 36(6) 939
/4/ Tom W.J., Debenedetti P.G., Biotechnol. Prog. 1991, 7, 403
/5/ Matson D.W., Petersen R.G., Smith R.; Advances in Ceramics 1987, 21, 1090

## Patentansprüche

1. Verfahren zur Herstellung von Partikeln bzw. Pulvern, insbesondere von Feststoffpartikeln, mit den Schritten:
- Bereitstellen eines Druckbehälters, der eine zu behandelnde Substanz bzw. Substanzmischung enthält,
- Auflösen eines kompressiblen Fluids in der vorgelegten Substanz bzw. Substanzmischung unter Druck bis zur Bildung einer Lösung,
- Entspannen der erhaltenen Lösung mittels einer Entspannungsvorrichtung derart, daß die Erstarrungstemperatur der Substanz bzw. Substanzmischung nach der Entspannungsvorrichtung unterschritten wird und eine Bildung von Partikeln stattfindet, und
- Abtrennen der gebildeten Partikeln aus dem Strom entkomprimierten, kompressiblen Fluids.

2. Verfahren nach Anspruch 1,
dadurch gekennzeichnet, daß der Druck während des Auflösens im Bereich von 5 bis 500 bar, insbesondere im Bereich von 10 bis 200 bar liegt

3. Verfahren nach Anspruch 1 oder 2,
dadurch gekennzeichnet, daß die nach dem Auflösen des kompressiblen Fluids erhaltene Lösung auf einer Temperatur gehalten wird, die im Bereich von bis zu 50 °K, bevorzugt 20 °K und besonders bevorzugt bis zu 10 °K über oder unter dem Schmelzpunkt der zu behandelnden Substanz bzw. Substanzmischung bei Atmosphärendruck liegt.

4. Verfahren nach einem der Ansprüche 1 bis 3,
dadurch gekennzeichnet, daß im Fall von Substanzen bzw. Substanzmischungen, welche sich beim Erhitzen unter Atmosphärendruck zersetzen, bevor sie schmelzen, die nach dem Auflösen des kompressiblen Fluids erhaltene Lösung auf einer Temperatur gehalten wird, die unterhalb der Zersetzungstemperatur der zu behandelnden Substanz bzw. Substanzmischung bei Atmosphärendruck liegt.

5. Verfahren nach einem der vorhergehenden Ansprüche,
dadurch gekennzeichnet, daß der Schmelzpunkt der zu behandelnden Substanz bzw. Substanzmischung mittels eines inkompressiblen Hilfsstoffs erniedrigt wird, der vor, während oder nach dem Auflösen des kompressiblen Fluids zugegeben wird.

6. Verfahren nach Anspruch 5,
dadurch gekennzeichnet, daß der inkompressible Hilfsstoff mit der zu behandelnden Substanz bzw. Substanzmischung ein niedrigschmelzendes Eutektikum bildet.

7. Verfahren nach einem der vorhergehenden Ansprüche,
dadurch gekennzeichnet, daß das kompressible Fluid ausgewählt wird aus der Gruppe Kohlenwasserstoffe mit 1 bis 6 C-Atomen, insbesondere Methan, Ethan, Propan, Butan, Pentan, n-Hexan, i-Hexan, Kohlendioxid, Freone, Stickstoff, Edelgase, gasförmige Oxide, z.B. N₂O, SO₂, Ammoniak, Alkohole mit 1 bis 4 C-Atomen, insbesondere Methanol, Ethanol, Isopropanol, n-Propanol, Butanol, halogenierte Kohlenwasserstoffe oder Mischungen der vorgenannten Stoffe.

8. Verfahren nach einem der vorhergehenden Ansprüche,
dadurch gekennzeichnet, daß die gebildeten Partikel fraktioniert abgetrennt werden.

9. Verfahren nach Anspruch 8,
dadurch gekennzeichnet, daß der Partikelstrom zur fraktionierten Abtrennung zunächst durch einen Sprühturm, dann durch einen Zyklon und schließlich durch einen Feinfilter, insbesondere durch einen Elektrofilter, geleitet wird.

10. Verfahren nach einem der vorhergehenden Ansprüche,
dadurch gekennzeichnet, daß die erhaltene Lösung durch eine Düse oder ein Ventil oder einen Diffusor oder eine Kapillare entspannt wird.

## Claims

1. A process for the production of particles or powders, in particular of solid particles, having the steps:
- provision of a pressure vessel which contains a substance or mixture of substances to be treated,
- dissolution of a compressible fluid under pressure in the substance or mixture of substances provided, until a solution forms,
- decompression of the resulting solution by means of a decompression apparatus in such a way that the temperature falls below the solidification point of the substance or mixture of substances downstream of the decompression apparatus, and formation of particles takes place, and
- removal of the particles which have formed from the stream of decompressed compressible fluid.

2. The process as claimed in claim 1,
characterized in that the pressure during the dissolution is in the range from 5 to 500 bar, in particular in the range from 10 to 200 bar.

3. The process as claimed in claim 1 or 2,
characterized in that the solution obtained after the dissolution of the compressible fluid is kept at a temperature which is in the region of up to 50 K, preferably 20 K, and particularly preferably up to 10 K, above or below the melting point under atmospheric pressure of the substance or mixture of substances to be treated.

4. The process as claimed in any of claims 1 to 3,
characterized in that in the case of substances or mixtures of substances which decompose on heating under atmospheric pressure before they melt, the solution obtained after dissolution of the compressible fluid is kept at a temperature which is below the decomposition temperature under atmospheric pressure of the substance or mixture of substances to be treated.

5. The process as claimed in any of the preceding claims,
characterized in that the melting point of the substance or mixture of substances to be treated is reduced by means of an incompressible auxiliary which is added before, during or after dissolution of the compressible fluid.

6. The process as claimed in claim 5,
characterized in that the incompressible auxiliary forms a low-melting eutectic with the substance or mixture of substances to be treated.

7. The process as claimed in any of the preceding claims,
characterized in that the compressible fluid is selected from the group of hydrocarbons with 1 to 6 C atoms, in particular methane, ethane, propane, butane, pentane, n-hexane, i-hexane, carbon dioxide, freons, nitrogen, inert gases, gaseous oxides, for example N₂O, SO₂, ammonia, alcohols with 1 to 4 C atoms, in particular methanol, ethanol, isopropanol, n-propanol, butanol, halogenated hydrocarbons or mixtures of the above-mentioned substances.

8. The process as claimed in any of the preceding claims,
characterized in that the particles which are formed are removed in fractions.

9. The process as claimed in claim 8,
characterized in that the particle stream is passed, for the fractional removal, first through a spray tower, then through a cyclone and finally through a fine filter, in particular through an electrostatic filter.

10. The process as claimed in any of the preceding claims,
characterized in that the resulting solution is decompressed through a nozzle or a valve or a diffuser or a capillary.

## Revendications

1. Procédé de fabrication de particules respectivement de poudre, en particulier de particules solides, comprenant les étapes consistant à :
- préparer un récipient sous pression qui contient une substance respectivement un mélange de substances à traiter,
- dissoudre sous pression un fluide compressible dans la substance respectivement dans le mélange de substances préparé(e) jusqu'à formation d'une solution,
- décompresser la solution obtenue au moyen d'un dispositif de décompression de telle façon que la température de solidification de la substance respectivement du mélange de substances soit dépassée vers le bas après le dispositif de décompression et qu'une formation de particules ait lieu, et
- séparer les particules formées du flux de fluide compressible décompressé.

2. Procédé selon la revendication 1,
caractérisé en ce que la pression pendant la dissolution se situe dans une plage de 5 à 500 bar, en particulier dans une plage de 10 à 200 bar.

3. Procédé selon la revendication 1 ou 2,
caractérisé en ce que la solution obtenue après la dissolution du fluide compressible est maintenue à une température qui se situe dans une plage de jusqu'à 50°K, de préférence de 20°K et de façon particulièrement préférée de jusqu'à 10°K au-dessus ou au-dessous du point de liquéfaction de la substance respectivement du mélange de substances à traiter à la pression atmosphérique.

4. Procédé selon l'une quelconque des revendications 1 à 3,
caractérisé en ce que dans le cas de substances respectivement de mélanges de substances qui se décomposent par chauffage sous pression atmosphérique avant de se liquéfier, la solution obtenue après dissolution du fluide compressible est maintenue à une température qui se situe en dessous de la température de décomposition sous pression atmosphérique de la substance respectivement du mélange de substances à traiter.

5. Procédé selon l'une quelconque des revendications précédentes,
caractérisé en ce que le point de fusion de la substance respectivement du mélange de substances à traiter est abaissé au moyen d'un adjuvant incompressible qui est ajouté avant, pendant ou après la dissolution du fluide compressible.

6. Procédé selon la revendication 5,
caractérisé en ce que l'adjuvant incompressible forme avec la substance respectivement le mélange de substances à traiter un système eutectique à bas point de fusion.

7. Procédé selon l'une quelconque des revendications précédentes,
caractérisé en ce que le fluide compressible est choisi dans le groupe des hydrocarbures comprenant de 1 à 6 atomes de carbone, en particulier le méthane, l'éthane, le propane, le butane, le pentane, le n-hexane, l'i-hexane, le gaz carbonique, le fréon, l'azote, les gaz rares, les oxydes gazeux, par exemple N₂O, SO₂, l'ammoniac, les alcools comprenant de 1 à 4 atomes de carbone, en particulier le méthanol, l'éthanol, l'isopropanol, le n-propanol, le butanol, les hydrocarbures halogénés ou des mélanges des produits précités.

8. Procédé selon l'une quelconque des revendications précédentes,
caractérisé en ce que les particules formées sont séparées par fractionnement.

9. Procédé selon la revendication 8,
caractérisé en ce que le flux de particules traverse pour la séparation par fractionnement d'abord une tour de pulvérisation puis un cyclone et enfin un filtre fin, en particulier un électrofiltre.

10. Procédé selon l'une quelconque des revendications précédentes,
caractérisé en ce que la solution obtenue est détendue dans une buse ou une soupape ou un diffuseur ou un tube capillaire.
